(19) Europäisches Patentamt
European Patent Office
Office européen des brevets

(11) **EP 4 484 932 A1**

(12) **DEMANDE DE BREVET EUROPEEN**

(43) Date de publication:
**01.01.2025 Bulletin 2025/01**

(21) Numéro de dépôt: **24184503.1**

(22) Date de dépôt: **25.06.2024**

(51) Classification Internationale des Brevets (IPC):
**G01N 21/64** (2006.01) **C12Q 1/6869** (2018.01)

(52) Classification Coopérative des Brevets (CPC):
**G01N 21/6408; G01N 21/6428**

(84) Etats contractants désignés:
**AL AT BE BG CH CY CZ DE DK EE ES FI FR GB GR HR HU IE IS IT LI LT LU LV MC ME MK MT NL NO PL PT RO RS SE SI SK SM TR**
Etats d'extension désignés:
**BA**
Etats de validation désignés:
**GE KH MA MD TN**

(30) Priorité: **27.06.2023 FR 2306747**

(71) Demandeurs:
- **Commissariat à l'Energie Atomique et aux Energies Alternatives**
  **75015 Paris (FR)**
- **Centre National de la Recherche Scientifique**
  **75016 Paris Cedex 16 (FR)**

- **Université Grenoble Alpes**
  **38400 Saint-Martin-d'Hères (FR)**

(72) Inventeurs:
- **CONSTANTIN, Olivier**
  **38054 GRENOBLE CEDEX 09 (FR)**
- **HOCEVAR, Moira**
  **38042 GRENOBLE (FR)**
- **MAILLEY, Pascal**
  **38054 Grenoble cedex 09 (FR)**
- **MONROY, Eva**
  **38054 GRENOBLE CEDEX 09 (FR)**

(74) Mandataire: **INNOV-GROUP**
  **209 Avenue Berthelot**
  **69007 Lyon (FR)**

(54) **DISPOSITIF DE DÉTECTION DE FLUORESCENCE PAR NANOPHOTODÉTECTEURS**

(57) L'invention concerne un dispositif de d'identification d'un marqueur fluorescent. Le dispositif de détection comporte des nanofils, chaque nanofil agissant comme un transducteur d'une lumière de fluorescence en un signal électrique. Une application visée est le séquençage d'un acide nucléique.

Fig. 3A

Fig. 3B

**Description**

## DOMAINE TECHNIQUE

Le domaine technique de l'invention est la détection de biomolécules par transduction optique effectuée par des nanofils.

## ART ANTERIEUR

Les dispositifs permettant de détecter des biomolécules peuvent être basés sur une détection optique. Les biomolécules à détecter sont préalablement marquées à l'aide d'un marqueur luminescent, de type fluorophore ou boite quantique (quantum dot). La détection est effectuée par un photodétecteur matriciel, de type imageur CCD, CMOS ou matrices de photodétecteurs à avalanches (APD : Avalanche Photodetectors).

Une application répandue du marquage fluorescent est le séquençage, en particulier le séquençage dit SBS (Sequencing by Synthesis). Selon cette méthode, une séquence de nucléotides mono-brin, disposée en aval d'une amorce, est amplifiée de façon à former un cluster (grappe). Lors du séquençage, une séquence complémentaire est progressivement synthétisée, par ajout successifs de bases nucléiques complémentaires de celles formant la séquence étudiée. Les bases complémentaires, sont ajoutées une par une, respectivement au cours de cycles. Après chaque ajout d'une base complémentaire, une image du cluster est réalisée. Tout ou partie des bases complémentaires est marquée par un label fluorescent. L'observation de la fluorescence au cours de chaque cycle permet d'identifier la base complémentaire ajoutée au cours dudit cycle. Ainsi, en réalisant une succession d'images de fluorescence, on peut décoder la séquence de nucléotides examinée.

La détection de fluorescence est effectuée par un photodétecteur, usuellement un photodétecteur matriciel. Cependant, les photodétecteurs présentent une matrice de détection dont le pas spatial est généralement supérieur à 1 μm. Par pas spatial, on entend la distance entre deux pixels adjacents. Un tel pas spatial est considéré comme trop élevé pour obtenir un dispositif permettant d'adresser un grand nombre de détections en parallèle. Ces dispositifs présentent également une sensibilité limitée : il est nécessaire de disposer d'une amplification suffisante pour obtenir un nombre suffisant de séquences identiques au niveau du cluster étudié, de façon à obtenir un signal exploitable.

Certains biocapteurs sont basés sur une détection de charges portées par les biomolécules cibles à détecter. Il s'agit par exemple de transistors à effet de champ. Ce type de dispositif permet d'effectuer une détection rapide, avec une bonne sensibilité. De plus, la technologie CMOS (semi-conducteur à oxyde métallique complémentaire) permet une fabrication de dispositifs comportant un grand nombre de capteurs. Il est ainsi possible d'effectuer un grand nombre d'analyses en parallèle. Cependant, ce type de capteur peut être sensible à des paramètres environnementaux affectant l'échantillon, par exemple pH ou température ou interactions avec des biomolécules non ciblées ou d'ions induisant des erreurs de détection. Le recours à ce type de biocapteurs dans des applications de séquençage d'ADN (acide désoxyribonucléique) suppose une certaine redondance de mesures, de façon à augmenter la robustesse des mesures. Les séquenceurs ADN basés sur des nanopores présentent les mêmes inconvénients.

Des capteurs photoniques basés sur des nanofils ont été développés à des fins de détection de fragments d'ADN. C'est par exemple le cas de la publication Sing, et al., « Silicon nanowire optical rectangular waveguide biosensor for DNA Hybridization », IEEE Photonics Technology Letters, 2018, 30(12) 1123-1126. Dans cette publication, on détecte une hybridation d'ADN en détectant une variation d'indice de réfraction lors de l'hybridation.

La publication Irrera, et al., "New génération of ultrasensitive label-free optical Si nanowire-based biosensors", ACS Photonics 2018, 5(2) 471-479 décrit un biocapteur à base de nanofils utilisés pour détecter la CRP (C-réactive protéine) dans du sérum humain.

Des capteurs mettant en oeuvre des matrices de nanofils ont également été développés, en particulier pour des applications visant des cellules photovoltaïques, comme décrit dans la publication Spies Maria et al « Nanowire photodetectors based on wurtzite semiconductor heterostructures" ou dans US2011253982. Des capteurs à base de nanofils pouvant être appliqués à l'imagerie ont été décrits dans Ziyuan Li et al « Review on III V Semiconductor nanowire array infrared photodetectors ».

Les applications liées au séquençage d'ADN supposent un codage par fluorescence, de façon à différencier les bases ajoutées. Dans les applications mettant en oeuvre un capteur, le codage est effectué soit par la longueur d'onde d'émission, soit par une intensité lumineuse au niveau de l'image acquise par le photodétecteur. US2022099575 décrit un capteur comportant des photodétecteurs élémentaires, formant des pixels. Les pixels intègrent un guide de lumière, et sont configurés pour permettre une analyse fluorescence en réflexion : le guide de lumière est disposé entre l'échantillon à analyser et le pixel permettant la détection de la lumière de fluorescence.

Les inventeurs ont conçu un dispositif d'analyse d'un échantillon compact et sensible, permettant de tirer profit des capacités de détection de fluorescence par des nanofils. Le dispositif peut permettre une analyse simultanée ou séquentielle de différentes biomolécules. Le dispositif permet de bénéficier de la sensibilité de détection et de la compacité conférée par les nanofils, tout en permettant une détection différenciée de bases nucléiques marquées par des marqueurs fluorescents.

## EXPOSE DE L'INVENTION

Un premier objet de l'invention est un dispositif d'identification d'un marqueur fluorescent, le marqueur fluorescent étant configuré pour émettre une lumière de fluorescence dans une bande spectrale de fluorescence, le dispositif comportant :

- un substrat, comportant au moins une première électrode;
- une structure multicouches, comportant au moins une deuxième électrode;
- des nanofils, s'étendant entre la première électrode et la deuxième électrode, parallèlement à un axe transversal;
- une couche d'encapsulation s'étendant autour des nanofils, entre le substrat et la structure multicouches, la couche d'encapsulation étant formée d'un matériau isolant ;
- la structure multicouches comportant :

  • une couche conductrice, formant chaque deuxième électrode;
  • une couche d'interface, électriquement isolante, recouvrant chaque deuxième électrode, chaque deuxième électrode étant interposée entre la couche d'interface et un nanofil, la couche d'interface étant délimitée par une surface de fonctionnalisation, la couche d'interface étant configurée pour être disposée entre un échantillon, comportant le marqueur fluorescent, et la deuxième électrode, de telle sorte que la surface de fonctionnalisation forme une interface entre le dispositif et l'échantillon ;
  • la structure multicouche étant telle que la deuxième électrode et la couche d'interface sont transparentes dans une bande spectrale de détection comportant la bande spectrale de fluorescence ;

le dispositif étant tel que :

- chaque nanofil comporte une jonction de type homojonction, ou une hétérojonction, ou une jonction Schottky entre la première électrode et la deuxième électrode ;
- la première électrode et la deuxième électrode sont configurées pour être raccordées à un circuit de détection;
- de telle sorte que chaque nanofil forme un nanophotodétecteur de la lumière de fluorescence lorsque le marqueur fluorescent est lié à la surface de fonctionnalisation, la lumière détectée par chaque nanofil induisant un signal électrique de détection dans le circuit de détection ;

le dispositif comportant une unité de traitement, programmée pour :

- • acquérir le signal de détection durant une période temporelle de détection ;
- • déterminer une caractéristique du signal de détection durant la période temporelle de détection ;
- • identifier le marqueur fluorescent en fonction de la caractéristique.

Selon une possibilité, le dispositif comporte une source de lumière, configurée pour émettre une lumière d'excitation dans la bande spectrale d'excitation du marqueur fluorescent.

Selon une possibilité:

- la source de lumière est configurée pour émettre la lumière d'excitation durant une période temporelle d'excitation ;
- la période temporelle de détection est postérieure à la période temporelle d'excitation.

Selon une possibilité:

- suite à la période temporelle d'excitation, l'intensité de la lumière de fluorescence suit une croissance puis une décroissance ;
- la caractéristique du signal de détection est représentative de la décroissance de l'intensité de la lumière de fluorescence.

Selon une possibilité la surface de fonctionnalisation est configurée pour capturer un brin formant une chaine d'oligonucléotides.

Selon une possibilité, chaque nanofil comporte :

- une homojonction de type pn ;
- ou une hétérojonction ;
- ou une jonction Schottky de type p-métal ou n-métal.

Selon une possibilité au moins un nanofil comporte une première partie et une deuxième partie, séparées par la jonction, la première partie étant formée d'un semi-conducteur dopé n, la deuxième partie étant formée d'un semi-conducteur dopé p, la jonction formant une homojonction ou une hétérojonction.

Selon une possibilité au moins un nanofil comporte une première partie et une deuxième partie, séparées par la jonction, la première partie étant formée d'un semi-conducteur, la deuxième partie, adjacente d'une électrode, étant formée d'un métal, la jonction formant une jonction Schottky.

Selon une possibilité, la première partie et la deuxième partie s'étendent, selon l'axe transversal, de part et d'autre de la jonction.

Selon une possibilité la deuxième partie entoure la première partie, autour de l'axe transversal. Selon une possibilité la surface de fonctionnalisation est segmentée en différents sites de capture, chaque site de capture étant configuré pour capturer un brin formant une chaine d'oligonucléotides..

Selon une possibilité :

- la couche d'interface comporte deux sous-couches empilées l'une sur l'autre, formant une sous-couche inférieure et une sous-couche supérieure, la sous-couche inférieure étant interposée entre la couche conductrice et la sous-couche supérieure ;
- la sous-couche supérieure comporte des puits, débouchant dans la sous-couche inférieure, chaque puits étant disposé face à un nanofil, chaque puits formant une partie de la surface de fonctionnalisation ;
- la surface de fonctionnalisation est segmentée au niveau de chaque puits, de façon que chaque puits forme un site de capture.

Selon une possibilité, le dispositif comporte plusieurs nanofils, s'étendant entre une même première électrode et une même deuxième électrode, les nanofils formant une grappe de nanofils.

Selon une possibilité, le dispositif comporte plusieurs grappes de nanofils (35), distantes les unes des autres, de telle sorte qu'un nanofil d'une grappe est plus proche d'un autre nanofil de ladite grappe que d'un autre nanofil d'une autre grappe, les nanofils d'une même grappe s'étendant entre une même première électrode et une même deuxième électrode.

Selon une possibilité, le dispositif comporte plusieurs nanofils, le dispositif étant tel que :

- plusieurs premières électrodes sont formées sur le substrat, et plusieurs deuxièmes électrodes sont formées sur la structure multicouche, chaque nanofil s'étendant entre une première électrode et une deuxième électrode ;
- chaque première électrode est reliée à une première unité d'adressage, configurée pour sélectionner au moins une première électrode ;
- chaque deuxième électrode est reliée à une deuxième unité d'adressage, configurée pour sélectionner au moins une deuxième électrode ;
- de telle sorte que le circuit de détection détecte un courant de détection induit par chaque nanofil s'étendant entre la première électrode et la deuxième électrode sélectionnées.

Un deuxième objet de l'invention est un procédé d'identification d'un marqueur fluorescent à l'aide d'un dispositif selon le premier objet de l'invention, le marqueur fluorescent étant susceptible d'émettre une lumière de fluorescence, dans la bande spectrale de détection, lorsqu'il est illuminé par la lumière d'excitation, la surface de fonctionnalisation étant configurée pour capturer un brin d'acide nucléique, le procédé comportant :

a) disposition d'un échantillon, comportant des acides nucléiques, au contact de la surface de fonctionnalisation ;
b) capture d'au moins un brin d'acide nucléique sur la surface de fonctionnalisation ;
c) ajout de bases nucléiques dans l'échantillon, au moins deux bases nucléiques différentes étant marquées par deux marqueurs fluorescents différents, l'échantillon comportant des principes actifs configurés pour permettre une hybridation d'une base nucléique sur le brin d'acide nucléique capturé sur la surface de fonctionnalisation ;
d) exposition de la surface de fonctionnalisation à une lumière d'excitation, dans une bande spectrale d'excitation d'au moins un marqueur fluorescent, ;
e) suite à l'étape d), détection d'un signal de détection aux bornes du circuit de détection, durant une période temporelle de détection ;
f) en fonction du signal de détection détecté lors de l'étape e), identification du marqueur fluorescent ;
g) réitération des étapes c) à f) de façon à hybrider progressivement des bases nucléiques le long du brin d'acide nucléique.

L'étape b) peut comporter une amplification de chaque brin d'acide nucléique capturé.

Selon une possibilité, les étapes b) à g) sont mises en oeuvre sur différents sites de capture répartis sur la surface de fonctionnalisation.

Selon une possibilité, l'étape f) comporte :

- détermination d'une caractéristique du signal de détection lors de la période temporelle de détection ;
- identification du marqueur fluorescent en fonction de la caractéristique.

Selon une possibilité:

- le marqueur fluorescent est choisi parmi plusieurs marqueurs fluorescents candidats ;
- l'étape f) comporte une sélection du marqueur fluorescent parmi les marqueurs fluorescents candidats en fonction de la caractéristique du signal de détection.

Selon une possibilité, l'étape f) comporte une estimation d'une dérivée temporelle du signal de détection.

Selon une possibilité, l'étape f) comporte une détection d'un niveau d'intensité ou une intégration du signal de détection durant au moins une période temporelle prédéterminée. Selon une possibilité:

- préalablement à l'étape a), chaque séquence de nucléotides est reliée à une séquence de calibration connue ;
- les étapes c) à f) sont mises en oeuvre de façon à hybrider les bases de la séquence de calibration, les étapes c) à f) formant une phase de calibration ;
- le signal de détection obtenu lors de chaque étape e) de la phase de calibration est utilisé pour calibrer une réponse du dispositif aux bases de la séquence de calibration.

Selon une possibilité:

- chaque marqueur fluorescent candidat émet une lumière de fluorescence selon une intensité de fluorescence croissante puis décroissante ;
- la décroissance de l'intensité de fluorescence de chaque marqueur fluorescent est caractérisée par une constante de décroissance ;
- les constantes de décroissance de deux marqueurs de fluorescence différents, sont différentes.

Selon une possibilité, suite à l'étape g), le procédé comporte une étape h) d'identification de la base d'acide nucléique hybridée.

Selon une possibilité,

- suite à l'étape h), le marqueur fluorescent est clivé et l'échantillon est rincé ;
- suite au rinçage, les étapes c) et h) sont réitérées, de manière à identifier une séquence de nucléotides formant le brin d'acide nucléique capturé.

L'invention sera mieux comprise à la lecture de l'exposé des exemples de réalisation présentés, dans la suite de la description, en lien avec les figures listées ci-dessous.

## FIGURES

Les figures 1A et 1B montrent les principaux composants d'un dispositif selon l'invention.

La figure 1C montre une distribution spatiale de sites de capture.

La figure 1D représente un exemple de dispositif selon l'invention, dans lequel les électrodes sont réparties selon un agencement matriciel.

La figure 1E montre un exemple de séquençage de trois séquences différentes, respectivement sur trois sites de capture.

La figure 2A montre des distributions temporelles d'émission de lumière de fluorescence de différents marqueurs fluorescents.

Les figures 2B et 2C montrent des exemples de caractérisation des distributions temporelles d'émission de lumière de fluorescence, la caractérisation étant réalisée durant une période temporelle de détection.

La figure 2D schématise différentes périodes temporelles de détection au cours de la décroissance de l'émission d'une lumière de fluorescence.

La figure 3A illustre une séquence de calibration.

La figure 3B montre différents niveaux d'intensité détectés lors d'une phase de calibration.

La figure 3C schématise des signaux de caractérisation successifs, respectivement lors d'un séquençage d'une séquence TTCG, avec clivage des marqueurs fluorescents au cours de chaque cycle.

La figure 3D schématise des signaux de caractérisation successifs, respectivement lors d'un séquençage d'une séquence TTCG, sans clivage des marqueurs fluorescents au cours de chaque cycle.

Les figures 4A à 4E schématisent des étapes de fabrication de nanofils selon un procédé dit ascendant.

La figure 5 représentent différents agencements possibles des nanofils.

Les figures 6A et 6B montrent deux structures différentes de nanofils.

La figure 7 montre différentes étapes de mise en oeuvre du procédé.

## EXPOSE DE MODES DE REALISATION PARTICULIERS

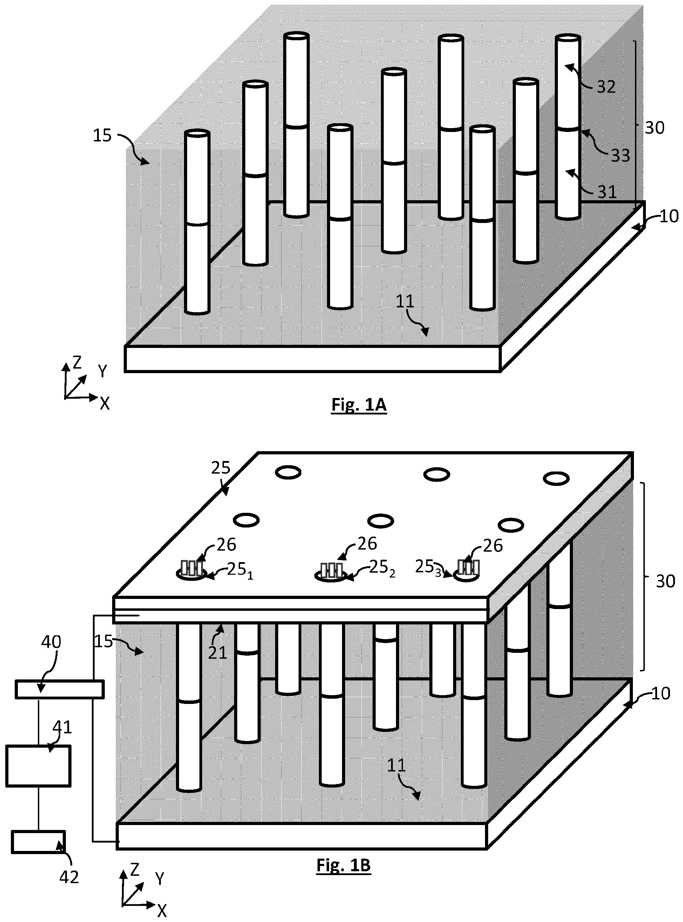

On a représenté, sur les figures 1A à 1E, un premier exemple de dispositif d'analyse 1 permettant une mise en oeuvre de l'invention. Le dispositif d'analyse 1 est configuré pour être placé au contact d'un échantillon 2. L'échantillon comporte par exemple un milieu liquide susceptible de contenir des brins d'acides nucléiques que l'on souhaite séquencer. Chaque brin est issu d'une fragmentation de matériel génétique, formant une librairie de brins. Chaque brin peut comporter un adaptateur au niveau de l'extrémité 3' et de l'extrémité 5'. De façon connue, l'adaptateur peut comporter un site de liaison d'amorce et éventuellement un index comportant un code d'identification de l'échantillon. Un des adaptateurs peut comporter une séquence dite de calibration, décrite par la suite.

Le dispositif comporte un substrat 10, formant ou comportant au moins une première électrode $11_c$. Dans l'exemple représenté sur la figure 1A, le substrat est un substrat de silicium cristallin, par exemple un substrat Si d'orientation cristalline (111). Le substrat 10 est délimité par une surface, dite première surface 11, comportant une première électrode $11_c$. Dans l'exemple de la figure 1A, la première surface 11 est formée de Si comportant des régions conductrices. Selon une autre possibilité le substrat 10 fait l'objet d'un dépôt d'une couche conductrice, par exemple du graphène, formant tout ou partie de la première surface 11.

Des nanofils 30 sont formés sur le substrat 10, et plus précisément à partir de la première surface 11. La première surface 11 s'étend selon un plan $P_{XY}$. Le plan $P_{XY}$ est défini par un axe longitudinal X et un axe latéral Y. Les axes X et Y sont sécants, et de préférence perpendiculaires l'un par rapport à l'autre. Les nanofils s'étendent parallèlement à un axe transversal Z sécant du plan $P_{XY}$. Dans les modes de réalisation décrits par la suite, l'axe transversal Z est perpendiculaire du plan

$P_{XY}$. La première surface 11 est conductrice au moins au niveau de l'intersection avec chaque nanofil 30. La totalité de la première surface 11 peut être conductrice.

Selon d'autres configurations, les nanofils peuvent être inclinés et non perpendiculaires au plan $P_{XY}$. Par exemple, si l'orientation cristalline du matériau formant le substrat 10 est (001), les nanofils peuvent croître selon une direction (111), donc en biais par rapport au plan $P_{XY}$.

Les nanofils 30 ont de préférence un diamètre compris entre 1 nm et 500 nm et une hauteur, selon l'axe transversal Z, comprise entre 300 et 1000 nm, voire 10000 nm.

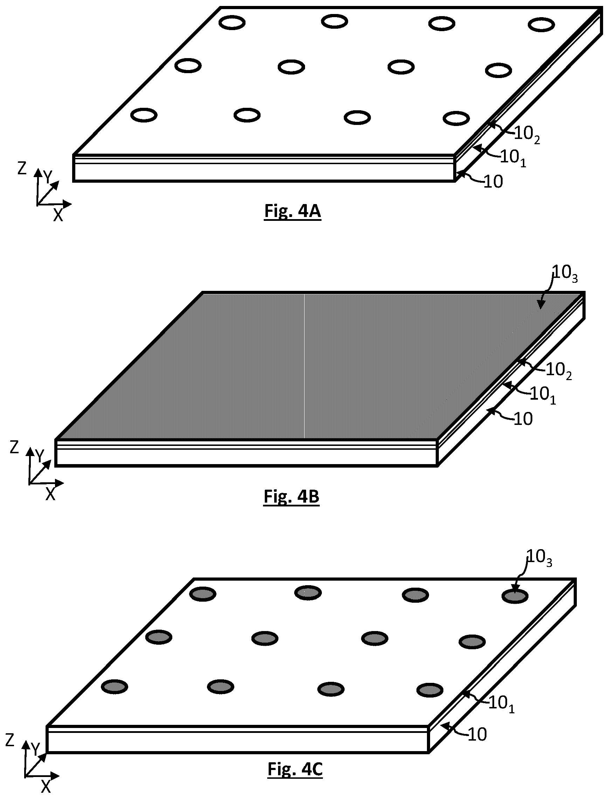

Les nanofils 30 peuvent être synthétisés directement sur le substrat 10, comme décrit en lien avec les figures 4A à 4E. Les nanofils peuvent être formés sur un autre substrat, puis transférés sur le substrat 10. Le transfert peut être effectué comme décrit dans la publication Valente, et al., « Light-Emitting GaAs Nanowires on a Flexible Substrate », Nano Lett 2018, 18(7) 4206-4213.

Les nanofils 30 s'étendent, à partir de la première surface 11, jusqu'à une deuxième surface 21 délimitant une structure multicouches 20. De même que la première surface 11, la deuxième surface 21 est conductrice au moins au niveau de l'intersection avec chaque nanofil 30. Dans l'exemple représenté sur la figure 1B, la deuxième surface 21 est formée à partir d'une couche 22 d'un matériau conducteur transparent dans une bande spectrale de détection décrite ci-après. Il peut par exemple s'agir d'ITO (oxyde d'indium étain).

Chaque nanofil est formé d'un ou plusieurs matériaux semi-conducteurs, et éventuellement d'un matériau métallique. Chaque nanofil comporte une jonction 33. Dans l'exemple représenté, la jonction 33 est une homojonction : chaque nanofil comporte une première partie 31, adjacente de la première surface 11, et une deuxième partie 32, adjacente de la deuxième surface 21. Les première et deuxième parties sont formées d'un même matériau semi-conducteur, avec respectivement deux dopages différents : ainsi, la première partie 31 et la deuxième partie 32 sont respectivement formés d'un même matériau semi-conducteur respectivement dopé n et p ou p et n. Dans l'exemple représenté, la première partie 31 est formée de GaAs (arséniure de gallium) dopé p et la deuxième partie est formée de GaAs dopé n. L'interface entre les deux parties forme la jonction pn 33.

De façon alternative, la jonction 33 peut être effectuée au niveau d'un métal disposé sur ou au contact de la surface 21 de façon à établir une jonction semi-conducteur - métal de type Schottky.

Chaque nanofil comporte au moins un semi-conducteur choisi parmi les matériaux des colonnes III et V, usuellement désignés par le terme matériaux III-V, par exemple, GaAs. Il peut de préférence s'agir d'un matériau de la colonne III et d'arsenic, par exemple InAs (arséniure d'indium). Dans l'exemple représenté, les nanofils 30 sont formés de GaAs. D'autres matériaux semi-conducteurs peuvent être envisagés, par exemple, et de façon non limitative, Si, InGaAs, AlGaAs, InGaP, InGaN, GaN, ZnSe, ZnS, ZnO, ZnCdO, ZnTe, CdSe, Ge, GeSn .

Entre la première surface 11 et la deuxième surface 21, les nanofils 30 sont noyés dans une couche d'encapsulation 15 formée d'un matériau isolant. La couche d'encapsulation 15 peut être formée d'un matériau de type PMMA (Polyméthacrylate de méthyle), BCB (Benzocyclobuthène) ou matériaux SOG (Spin-On Glass) constitués principalement d'oxydes de silicium et d'autres additifs chimiques pour conférer des propriétés spécifiques telles que l'adhérence et la stabilité thermique. La couche d'encapsulation 15 peut être déposée par spin-coating (dépôt à la tournette).

La couche d'encapsulation 15 est de préférence formée suite à la croissance des nanofils, préalablement au dépôt d'une deuxième couche conductrice 22, délimitée par la deuxième surface 21, et destinée à former des deuxièmes électrodes $21_c$. Les deuxièmes électrodes $21_c$ sont formées, au niveau de la deuxième surface 21, à partir de la couche conductrice 22. La couche conductrice 22 peut être structurée de façon qu'au niveau de la deuxième surface 21, plusieurs deuxièmes électrodes $21_c$ soient électriquement isolées les unes des autres. Ainsi, la couche conductrice peut comporter des ouvertures ou des matériaux isolant délimitant les électrodes $21_c$. Cela permet une détection différenciée au niveau de chaque nanofil.

Outre la couche conductrice 22, la structure multicouches 20 comporte une couche d'interface 23, adjacente de la couche conductrice 22. La couche d'interface 23 est transparente dans la bande spectrale de détection. La couche d'interface 23 peut par exemple être formée d'une couche d'un polymère, par exemple le PMMA (Polyméthylméthacrylate). La couche d'interface 23 est électriquement isolante, en particulier au niveau de l'interface avec la couche conductrice 22. La couche d'interface 23 est de préférence plane.

La couche d'interface 23 est destinée à former une interface entre la couche conductrice 22, formant les électrodes $21_c$, et l'échantillon 2. Ainsi, la couche d'interface 23 s'étend entre la couche conductrice 22 et l'échantillon 2. Un aspect important du dispositif est que l'échantillon n'est pas en contact direct avec les nanofils. Il est isolé de ces derniers par la couche d'interface 23.

La couche d'interface 23 est par exemple une couche mince formée de $SiO_2$ ou de PMMA. La surface de la couche d'interface 23, destinée à être en contact avec l'échantillon est une surface, dite surface de fonctionnalisation 25, fonctionnalisée par des espèces biologiques de capture 26. Il est important que la couche d'interface 23 soit formée d'un matériau présentant une autofluorescence aussi faible que possible dans la bande spectrale de détection.

La surface de fonctionnalisation 25, qui forme une interface entre la structure multicouches 20 et l'échantillon 2 à analyser, est destinée à être fonctionnalisée par des espèces de capture 26. Par espèce de capture, on entend une espèce configurée pour capturer une biomolécule d'intérêt, et plus précisément une séquence de nucléotides à analyser.

Selon une possibilité, l'espèce de capture est formée d'oligonucléotides configurés pour se greffer à l'acide nucléique à analyser. Elle peut notamment comporter une chaine de base de nucléotides complémentaire de tout ou partie des adapteurs reliés aux brins à caractériser. La liaison entre l'espèce de capture 26 et chaque brin d'oligonucléotides à analyser, ou plus précisément un adaptateur de chaque brin d'oligonucléotides à analyser, s'effectue alors par hybridation. La couche d'interface 23 peut être nanostructurée, de façon que des nanopuits 27 soient formés à l'interface entre la structure multicouches 20 et l'échantillon à analyser. Le diamètre ou la plus grande diagonale des nanopuits peut être comprise entre 70 nm et 700 nm. Les nanopuits 27 peuvent par exemple être agencés de façon matricielle, ou, de façon plus générale, selon un motif prédéterminé. La surface de fonctionnalisation est alors fonctionnalisée au niveau de chaque nanopuits 27, les espaces entre chaque puits n'étant pas fonctionnalisés. La formation des nanopuits peut être obtenue par amincissement local de la couche d'interface 23.

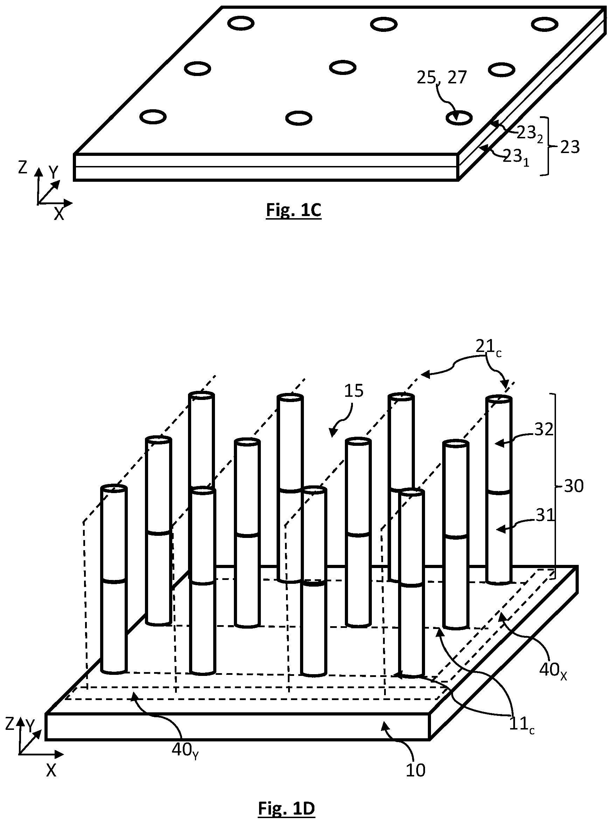

Selon une possibilité, représentée sur la figure 1C, la couche d'interface 23 comporte deux sous-couches superposées $23_1$ et $23_2$. La couche d'interface comporte une sous-couche inférieure $23_1$ interposée entre la couche conductrice 22 et une sous-couche supérieure $23_2$. Les nanopuits sont formés par amincissement local de la sous-couche supérieure $23_2$, de façon que les nanopuits débouchent dans la sous-couche inférieure $23_1$. La sous-couche supérieure $23_2$ peut être formée d'un matériau non fonctionnalisable, par exemple un matériau anti-biofouling (anti-encrassement biologique), par exemple un matériau hydrophobe. Une telle structuration permet que la fonctionnalisation de la surface de fonctionnalisation 25 soit effectuée uniquement au niveau des nanopuits 27, sur la sous-couche inférieure $23_1$. Chaque nanopuits 27 forme ainsi un site de capture d'un brin d'ADN.

De façon plus générale, la surface de fonctionnalisation 25 peut être fonctionnalisée selon un motif de fonctionnalisation prédéterminé. En dehors du motif de fonctionnalisation, la surface de fonctionnalisation n'est pas fonctionnalisée

La fonctionnalisation peut être effectuée par un traitement de la surface de fonctionnalisation 25, par exemple un traitement de surface plasma/oxygène. Lorsque la couche d'interface 23 est formée de PMMA, un traitement plasma/oxygène permet de former des fonctions carboxyles. Les espèces de capture 26 peuvent être greffées sur la surface de fonctionnalisation par liaison covalente. A cette fin, les espèces de capture comportent une fonction, par exemple une fonction amine, de façon à former une liaison covalente avec les fonctions de la surface de fonctionnalisation 25. Il peut par exemple s'agir d'une liaison obtenue par greffage d'une fonction Thiol, au niveau de l'espèce de capture, sur une fonction Amine présente au niveau de la surface de fonctionnalisation.

Selon une possibilité, chaque séquence à analyser comporte une fonction Thiol, par exemple au niveau de l'extrémité 3', de façon à former une liaison covalente avec la surface de fonctionnalisation. Dans ce cas, la surface de fonctionnalisation fait office d'espèce de capture.

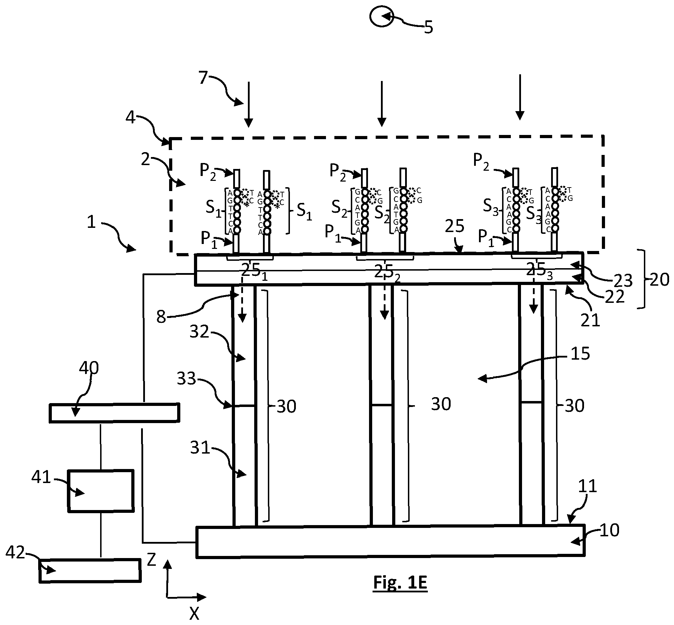

Comme représenté sur les figures 1B et 1E, le dispositif comporte un circuit de détection 40, dont une première borne est raccordée à une première électrode $11_c$, sur le substrat 10, et une deuxième borne est raccordée à une deuxième électrode $21_c$, sur la structure multicouches 20.

Le circuit de détection 40 permet de mesurer la différence de potentiel, ou un courant électrique, entre la première électrode $11_c$ et la deuxième électrode $21_c$.

Comme décrit en lien avec les figures 1A à 1C, chaque nanofil s'étend entre une première extrémité, sur une première surface 11 du substrat 10 et une deuxième extrémité, sur une deuxième surface 21 de la structure multicouche 20. La première surface 11 et la deuxième surface 21 sont conductrices au moins au niveau de chaque intersection avec un nanofil. Ainsi, au niveau de chaque intersection avec un nanofil, la première surface comporte une première électrode $11_c$ et la deuxième surface comporte une deuxième électrode $21_c$. Dans l'exemple représenté sur les figures 1A à 1B, la première surface 11 et la deuxième surface 21 sont formées d'un matériau conducteur. Elles sont conductrices selon toute leur aire.

Les première et deuxième surfaces peuvent être structurées, et comporter différentes électrodes $11_c$, $21_c$ isolées les unes des autres. Sur la figure 1D, chaque électrode est matérialisée par une ligne en pointillés. Sur la première surface 11, chaque première électrode $11_c$ décrit une ligne, parallèle à l'axe longitudinal X. Sur la deuxième surface 21, chaque deuxième électrode $21_c$ décrit une colonne, parallèle à l'axe latéral Y. Chaque nanofil est fonctionnel lorsque les électrodes $11_c$, $21_c$, entre lesquelles il s'étend, sont polarisées. La structuration des électrodes en lignes / colonnes permet de sélectionner les nanofils fonctionnels, ces derniers s'étendant entre deux électrodes polarisées : selon cet agencement, les nanofils reliés à une ligne et à une colonne polarisées sont fonctionnels. Par nanofil fonctionnel, on entend un nanofil polarisé pour effectuer une détection de photons de fluorescence.

Plusieurs lignes et/ou plusieurs colonnes peuvent être polarisées simultanément ou successivement. Le circuit de détection 40 comporte :

- une unité d'adressage $40_X$, destinée à polariser tout ou partie des premières électrodes $11_c$, parallèles à l'axe X,
- et une unité d'adressage $40_Y$ destinée à polariser tout ou partie des deuxièmes électrodes $21_c$ parallèles à l'axe Y.

Les parties fonctionnalisées de la surface de fonctionnalisation forment des sites de capture de séquences de nucléotides à caractériser.

Comme précédemment indiqué, chaque espèce biologique de capture 26 est configurée pour capturer une espèce biologique d'intérêt, en particulier une séquence de nucléotides à analyser.

De préférence, après qu'une séquence de nucléotides a été capturée sur un site de capture, cette dernière fait l'objet d'une amplification, de façon à obtenir, au niveau d'un même site de capture, une pluralité de répliques identiques, aux erreurs d'amplification près, de la séquence de nucléotides capturée.

L'amplification de chaque séquence capturée peut être de type «amplification par pont », usuellement désigné par le terme « bridge amplification », et usuellement mise en oeuvre dans des dispositifs proposés par la société Illumina. Lorsqu'on met en oeuvre une amplification par pont, la surface de fonctionnalisation porte des espèces de capture configurées pour se greffer aux adaptateurs des séquences capturées.

Sur la figure 1E, on a représenté trois séquences $S_1$, $S_2$ et $S_3$ respectivement capturées, puis amplifiées, sur trois sites de capture $25_1$, $25_2$ et $25_3$.Chaque séquence de nucléotides à caractériser est par exemple obtenue selon une méthode de préparation d'une librairie de fragments d'ADN courts, dont la longueur est typiquement de 300 bases ou 150 bases.

D'une façon générale, le séquençage comporte plusieurs cycles, au cours desquels des bases complémentaires sont progressivement hybridées le long de chaque séquence à caractériser. De façon connue, au cours de chaque cycle, les séquences à caractériser baignent dans un milieu réactionnel comportant des bases marquées par un marqueur fluorescent et une enzyme hybridase. L'hybridation s'effectue progressivement le long de chaque brin, base par base, dans un sens prédéterminé, par exemple de l'extrémité libre vers l'extrémité liée à l'espèce de capture.

Selon une possibilité, les marqueurs fluorescents sont couplés à un terminateur, ce qui empêche l'hybridation de deux bases consécutives au cours d'un même cycle. Le milieu réactionnel comporte alors un mélange de bases, chaque base de même type étant marquée par un même marqueur fluorescent. Deux bases de types différents sont respectivement marquées par des marqueurs fluorescents différents. A la suite de chaque hybridation, une détection du marqueur fluorescent est effectuée après rinçage, ce qui permet d'identifier la base qui s'est hybridée au cours du cycle. Suite à la détection, au moins le terminateur subit un clivage. Le marqueur fluorescent peut également subir un clivage. Le milieu est ensuite rincé et un autre cycle est initié. Les cycles se répètent jusqu'au séquençage complet des séquences greffées au niveau des différents sites de capture. Parmi les quatre bases différentes mises en oeuvre, une base peut ne pas être marquée.

Selon une autre possibilité, au cours de chaque cycle, les séquences baignent successivement dans des bains, chaque bain comportant un seul type de base. Chaque base du même type est marquée par un même marqueur fluorescent. Selon cette possibilité, il est possible, mais pas nécessaire que deux bases de types différents soient respectivement marquées par des marqueurs différents. En effet, l'identification de chaque type de base est effectuée par chaque bain. Entre chaque bain, un rinçage est effectué et une détection d'une fluorescence au niveau de chaque site de capture est effectuée. Selon cette possibilité, les marqueurs fluorescents ne comportent pas forcément de terminateurs. Ainsi, une pluralité de bases successives identiques peuvent être hybridées par des bases complémentaires du milieu réactionnel. Il en résulte un signal de fluorescence dont l'intensité augmente en fonction du nombre de bases successives hybridées.

Suite à la détection d'un signal de fluorescence, les marqueurs fluorescents marquant chaque base hybridée au cours du cycle peuvent être clivés. Comme décrit par la suite, le clivage n'est pas nécessaire.

Ainsi, au niveau de chaque site de capture, chaque cycle comporte :

- l'ajout d'un milieu réactionnel comportant des bases et une enzyme hybridase (enzyme polymérase) :

  ◦ soit sous la forme d'un mélange de bases de différents types, auquel cas les marqueurs fluorescents comportent un terminateur ;
  ◦ soit sous la forme de bains successifs, chaque bain comportant des bases de même type, auquel cas les marqueurs fluorescents peuvent ne pas comporter de terminateur ;

- un rinçage ;
- une détection, par les nanofils, d'une fluorescence (ou d'une absence de fluorescence)
- suite à la détection d'une fluorescence, un éventuel clivage des éventuels terminateurs et éventuellement des marqueurs fluorescents, suivi d'un rinçage.

Entre chaque cycle, le milieu réactionnel est lavé et renouvelé. Chaque cluster est excité par une source de lumière 5, dans une bande d'excitation du marqueur fluorescent ou de chaque marqueur fluorescent. La source de lumière est configurée pour émettre une lumière d'excitation dans une durée courte, de l'ordre de la ns. Il peut par exemple s'agir d'une source de lumière laser. Cela permet de générer, au niveau de chaque cluster, un signal de fluorescence, qui dépend de la base ajoutée (A, C, T ou G dans le cas d'un brin d'ADN).

Un aspect important de l'invention, décrit par la suite, consiste à traduire le signal de fluorescence en un signal électrique dépendant du marqueur fluorescent, ce qui permet d'obtenir, au cours d'un cycle, un signal électrique dépendant de la base ajoutée au cours du cycle.

Il s'agit d'une approche sensiblement différente de l'approche de séquençage basée sur une formation d'une image de l'échantillon, chaque base étant identifiée par un code couleur et/ou par un niveau d'intensité.

L'invention tire profit de la capacité d'un nanofil à détecter un signal optique, dans une bande spectrale prédéfinie, et à convertir le signal optique en un signal électrique de détection. Ainsi, le dispositif 1 est basé sur une détection optique de l'hybridation d'une base sur une séquence à analyser, induisant une réponse électrique du dispositif.

Sur la figure 1E, on a représenté une vue en coupe du dispositif, après amplification de séquences .On suppose qu'au niveau de chaque site de capture, une seule séquence a été capturée (séquence $S_1$ au niveau du site $25_1$, séquence $S_2$ au niveau du site de capture $25_2$...). Chaque séquence capturée a ensuite fait l'objet d'une amplification par pont. Après l'amplification, au niveau de chaque site de capture s'étendent des séquences identiques, formées, au niveau de leur extrémité 3' à partir d'un adaptateur $P_1$. Les séquences comportent, à leur extrémité 5' un adaptateur $P_2$. L'adaptateur $P_1$ fait partie des espèces de capture 26 greffées, par liaison covalente, sur la surface de fonctionnalisation. L'adaptateur $P_2$ de chaque séquence est libre.

Le milieu réactionnel 2 est disposé dans une chambre fluidique 4, au contact de la couche d'interface 23. Les séquences à caractériser et le milieu réactionnel sont isolés des nanofils 30 par la couche d'interface 23. Dans cet exemple, on se place dans la configuration selon laquelle le milieu réactionnel comporte différents types de bases marquées par des marqueurs fluorescents.

Au cours de chaque cycle, une source de lumière 5 illumine la surface de fonctionnalisation 25, dans une bande spectrale d'excitation centrée sur une longueur d'onde d'excitation de fluorescence d'un marqueur fluorescent.

On se réfère à la séquence $S_1$, capturée et amplifiée sur le site de capture $25_1$. Au cours d'un premier cycle, une base T a été hybridée. La figure 1E représente un deuxième cycle, au cours duquel une base C marquée par un marqueur fluorescent C*, représenté par le symbole *, s'hybride à une base G de la séquence. L'objectif du dispositif est de détecter le marqueur fluorescent C*, par le biais d'une transduction de la lumière de fluorescence.

La source de lumière 5 génère une lumière d'excitation 7, qui se propage à travers l'échantillon 2 jusqu'à la surface de fonctionnalisation 25. Sous l'effet de l'illumination à la longueur d'onde d'excitation une lumière de fluorescence 8 est émise par le marqueur fluorescent C*, dans une longueur d'onde de fluorescence, plus élevée que la longueur d'onde d'excitation. Des photons de fluorescence sont émis, formant la lumière de fluorescence 8. Du fait de la transparence de la couche d'interface 23, ou du matériau 22 constituant l'électrode $21_c$, certains photons de fluorescence se propagent à travers un nanofil 30. Lorsqu'un photon de fluorescence est absorbé au niveau de la jonction 33, des paires électrons/trous sont formées. Sous l'effet de la différence de potentiel entre la première partie 31 et la deuxième partie 32, les électrons se propagent à travers la portion dopée n, vers le potentiel le plus élevé tandis que les trous se propagent en sens inverse à travers la portion dopée p. Il en résulte une augmentation du courant électrique circulant dans le circuit de détection 40.

Le choix du matériau semi-conducteur formant le nanofil 30 dépend de la bande spectrale d'absorption dudit matériau, cette dernière devant à la fois contenir la longueur d'onde de fluorescence, et, de préférence, ne pas contenir la longueur d'onde d'excitation du marqueur fluorescent. Le nanofil agit ainsi en tant que nanophotodétecteur de la lumière de fluorescence, tout en n'étant pas sensible à la longueur d'onde d'excitation. Chaque nanofil forme ainsi un filtre vis-à-vis de la longueur d'onde d'excitation. Eventuellement, une couche absorbante peut être ajoutée entre la surface de fonctionnalisation 25 et la couche conductrice 22 pour réduire la largeur spectrale de la réponse des nanofils, par exemple une couche d'oxyde de zinc déposée par pulvérisation. Lorsque le matériau semi-conducteur est GaAs, le marqueur fluorescent peut être Cy3 (Cyanine) : longueur d'onde d'excitation 540nm et longueur d'onde d'émission comprise entre 555 et 600nm.

De préférence, le diamètre des nanofils est contrôlé de façon à conférer une sensibilité dans une bande spectrale étroite. Par bande spectrale étroite, on entend une largeur de bande typiquement de quelques dizaines de nm, et de préférence inférieure à 100 nm. La largeur de bande correspond à la largeur à mi-hauteur du pic d'absorption sur le spectre d'absorption. La largeur de bande de détection est par exemple de l'ordre de 50 nm. Il est ainsi possible d'ajuster la bande spectrale de détection, de façon qu'elle comprenne la longueur d'onde de fluorescence du marqueur fluorescent et qu'elle ne comprenne pas la longueur d'onde d'excitation du marqueur fluorescent. La faculté de nanofils à former un photodétecteur sélectif en longueur d'onde a été décrite dans Mokkapati et al., « Optical design of nanowire absorbers for wavelength selective photodetectors », Sci. Rep. 2015, 5 15339.

La sensibilité spectrale de détection peut être ajustée par l'incorporation de puits quantiques ou de boites quantiques (quantum dots) au niveau de la jonction 33. Cela permet d'obtenir une jonction 33 dont la composition est différente par rapport au reste du nanofil. La longueur d'onde détectée correspond alors à la longueur d'onde du gap défini par le puits ou la boîte quantique.

Chaque nanofil forme ainsi un nanophotodétecteur. Au cours de chaque cycle, il est nécessaire non seulement de détecter une lumière de fluorescence, mais également d'identifier le marqueur fluorescent ayant généré la lumière de fluorescence, afin d'identifier la base à laquelle il était lié. Dans cet exemple, on suppose que les bases nucléiques contenues dans le milieu réactionnel sont respectivement marquées par un marqueur fluorescent A*, G*, C*. On suppose que les bases T ne sont pas marquées par un marqueur fluorescent. Au cours de chaque cycle, on détermine :

- soit l'absence d'une lumière de fluorescence, ce qui se traduit par une absence de variation du courant résultant des nanofils : cela correspond à une hybridation d'une base T ;
- soit une détection d'une lumière de fluorescence, en discriminant les marqueurs A*, G* et C*.

Un aspect important de l'invention est que la discrimination est effectuée par une caractérisation du courant électrique de détection, résultant des nanofils.

Afin de permettre une discrimination de la lumière de fluorescence respectivement émise par chaque marqueur fluorescent, chaque marqueur de fluorescence utilisé présente un ou plusieurs paramètres d'émission différent l'un de l'autre. Par paramètre d'émission, on entend un paramètre caractérisant une distribution temporelle de l'intensité de fluorescence émise par le marqueur fluorescent. Il peut par exemple s'agir de l'intensité maximale $I_{max}$ de la distribution et/ou du temps de décroissance de la lumière de fluorescence. Le temps de décroissance de la lumière de fluorescence correspond à une durée durant laquelle l'intensité de fluorescence décroît entre deux niveaux prédéterminés, par exemple entre l'intensité maximale $I_{max}$ et un certain pourcentage de l'intensité maximale, par exemple 50% ou 80%. La décroissance de l'intensité de fluorescence peut suivre une variation de type $e^{\frac{-t}{\tau}}$, où t correspond au temps et $\tau$ est une constante de décroissance. Dans ce cas, le temps de décroissance peut être caractérisé par la constante de décroissance $\tau$.

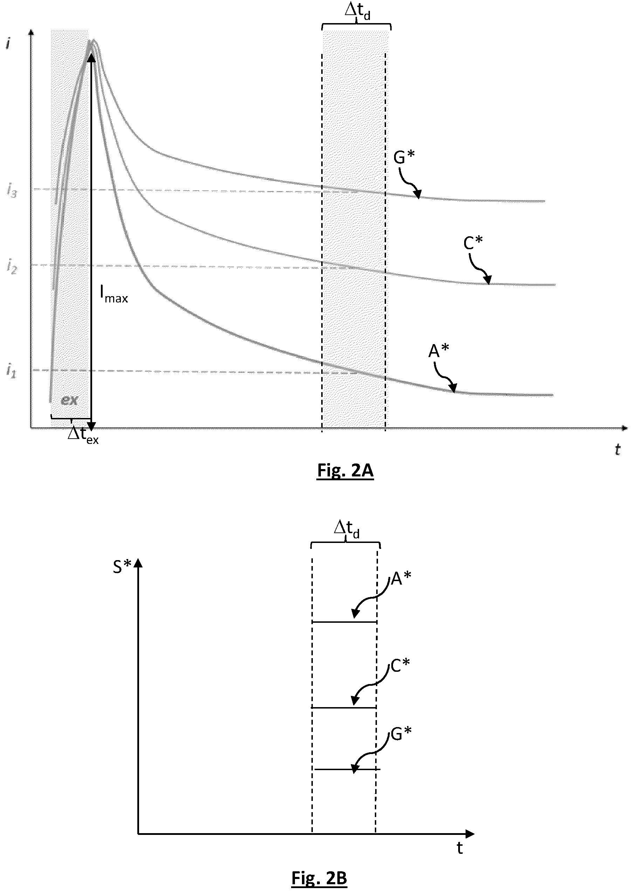

Sur la figure 2A, on a schématisé trois distributions temporelles de l'intensité de fluorescence des trois marqueurs fluorescents G*, C* et A*, marquant respectivement les bases G, C et A Dans cet exemple, l'intensité maximale $I_{max}$ est considérée comme identique pour ces trois marqueurs fluorescents. Cependant, la décroissance est plus rapide pour A* par rapport à C*, et pour C* par rapport à G*. Ainsi, $\tau(A^*) > \tau(C^*) > \tau(G^*)$. $t(A^*)$ désigne la constante de décroissance de A* et de façon analogue pour $\tau(C^*)$ et $\tau(G^*)$.

On désigne par S un signal de détection, résultant du circuit de détection 40, formé à partir des charges collectées au niveau de chaque nanofil. Le dispositif comporte une unité de traitement 41, permettant une caractérisation du signal de détection, de façon à identifier le marqueur fluorescent ayant généré la lumière de fluorescence. L'unité de traitement 41 peut comporter un microprocesseur 41, relié à une mémoire 42 dans laquelle des instructions sont mémorisées. L'unité de traitement forme un signal de caractérisation S* à partir du signal de détection.

Sur la figure 2A, on a représenté :

- une période temporelle d'excitation $\Delta t_{ex}$, qui correspond à l'impulsion lumineuse émise par la source de lumière 5. La période temporelle d'excitation $\Delta t_{ex}$ peut être par exemple de quelques dizaines de ps ou de l'ordre de la ns.
- une période temporelle de détection $\Delta t_d$, durant laquelle le signal de détection S est caractérisé, de façon à identifier le marqueur fluorescent ayant généré une lumière de fluorescence, ou l'absence de marqueur fluorescent. La période temporelle de détection $\Delta t_d$ peut être par exemple de 100 ps à 1 ns ou de quelques ns. La période de détection s'étend de préférence durant la décroissance de la lumière de fluorescence émise par le marqueur fluorescent.

L'émission de lumière de fluorescence permet d'obtenir un signal de détection exploitable pendant une durée de fluorescence de l'ordre de la ou plusieurs ns.

La figure 2B illustre un premier exemple de réalisation, dans lequel le signal de caractérisation correspond à une valeur absolue de la dérivée temporelle de l'intensité du courant électrique résultant de chaque nanofil. Ainsi, $S(t) = \left| \frac{di(t)}{dt} \right|$. On observe que :

- le signal de caractérisation est maximal lorsque le marqueur fluorescent à l'origine de la fluorescence est A* ;
- le signal de caractérisation est minimal lorsque le marqueur fluorescent à l'origine de la fluorescence est G* ;
- le signal de caractérisation est compris entre la valeur maximale et la valeur minimale lorsque le marqueur fluorescent à l'origine de la fluorescence est C*.

Une absence de variation du signal de détection permet de conclure qu'aucune fluorescence n'a été détectée. Cela traduit une hybridation d'une base T sur la séquence à caractériser.

Le mode de réalisation décrit en lien avec la figure 2B, basé sur une mesure différentielle de l'intensité de fluorescence, présente l'avantage d'être peu sensible à la variabilité du nombre de séquences respectivement amplifiées sur chaque site de capture.

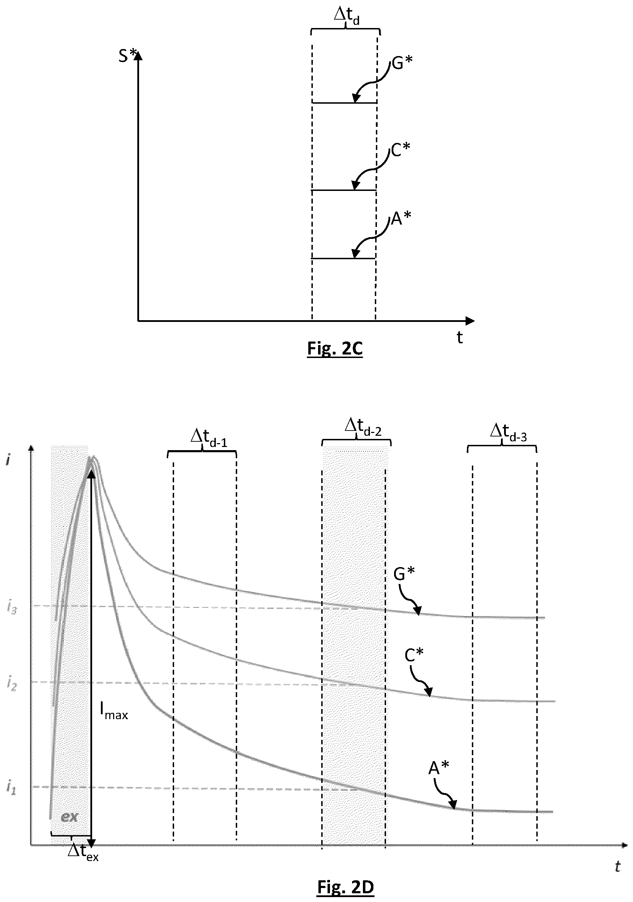

Dans un deuxième exemple, représenté sur la figure 2C, l'unité de traitement est programmée pour déterminer un signal de caractérisation S* qui correspond à une intégrale du signal de détection.

$$S^* = \int_{\Delta td}^{\square} S(t)dt,$$

On observe que :

- le signal de caractérisation est maximal lorsque le marqueur fluorescent à l'origine de la fluorescence est G* ;

- le signal de caractérisation est minimal lorsque le marqueur fluorescent à l'origine de la fluorescence est A* ;
- le signal de caractérisation est compris entre la valeur maximale et la valeur minimale lorsque le marqueur fluorescent à l'origine de la fluorescence est C*.

Lorsque le signal de caractérisation correspond à une intégration du signal de détection, le niveau atteint par le signal de caractérisation permet une discrimination entre les marqueurs fluorescents G*, A* et C*. Il en est de même lorsque le signal de caractérisation est une moyenne ou une médiane ou résultant d'un autre indicateur statistique représentatif de l'intensité du signal de détection durant la période de détection.

Cependant, le nombre de séquences à caractériser immobilisées sur la surface de fonctionnalisation, à l'aplomb d'un nanofil ou d'un même site de capture peut être variable, ainsi que le nombre d'erreurs d'amplification. Ainsi, le nombre de séquences identiques répliquées par amplification sur chaque site de capture peut varier d'un site de capture à un autre site de capture. Or, l'intensité du courant photoinduit dépend du nombre de marqueurs fluorescents marquant des bases hybridées au cours d'un même cycle. Pour adresser cette difficulté, une séquence de calibration $S_c$ peut être greffée sur chaque séquence à caractériser, de façon à établir les niveaux d'intensités respectivement associés à chaque marqueur fluorescent.

La séquence de calibration $S_c$ correspond à une suite de bases disposées dans un ordre connu, et dont on connaît l'emplacement dans l'adaptateur. Par exemple, la séquence de calibration est disposée à un emplacement connu dans l'adaptateur $P_2$ destiné à rester libre après l'amplification. Le recours à une séquence de calibration est particulièrement utile lorsque le signal de caractérisation correspond au signal de détection à un instant donné, ou comporte une intégrale du signal de détection.

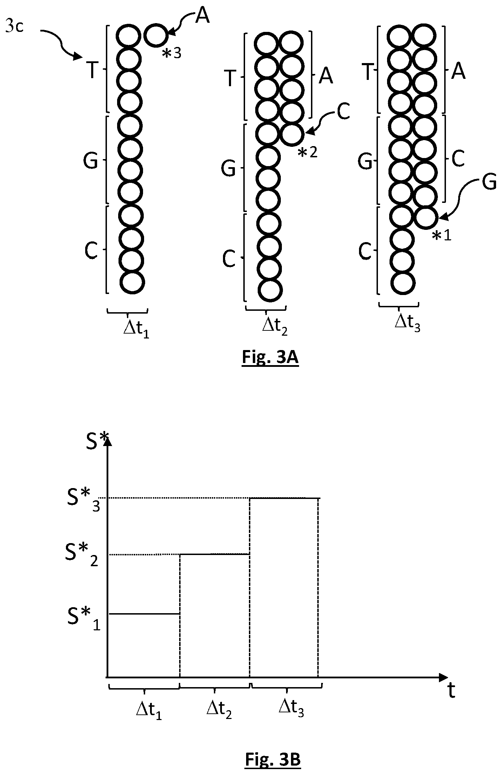

La figure 3A illustre un exemple de séquences de calibration, composée d'une succession de 4 bases T, 4 bases G et 4 bases C. Dans cet exemple, la séquence de calibration fait partie de l'adaptateur $P_2$. Le signal de caractérisation est une intégrale du signal de détection pendant une période temporelle prédéterminée. Lors du séquençage de la séquence de calibration :

- au cours d'une première période $\Delta t_1$, qui correspond à une période durant laquelle des bases complémentaires A sont successivement hybridées aux bases T de la séquence de calibration, l'intensité du signal de caractérisation correspond à un premier niveau $S^*_1$. Le marqueur fluorescent A* est symbolisé par *1 sur la figure 3A.
- au cours d'une deuxième période $\Delta t_2$, qui correspond à une période durant laquelle des bases complémentaires C sont successivement hybridées aux bases G de la séquence de calibration, l'intensité du signal de caractérisation correspond à un deuxième niveau $S^*_2$. Le marqueur fluorescent C* est symbolisé par *2 sur la figure 3A.
- au cours d'une troisième période $\Delta t_3$, qui correspond à une période durant laquelle des bases complémentaires G sont successivement hybridées aux bases C de la séquence de calibration, l'intensité du signal de caractérisation correspond à un troisième niveau $S^*_3$. Le marqueur fluorescent G* est symbolisé par *3 sur la figure 3A.

La figure 3B montre l'évolution du signal de caractérisation lors des périodes $\Delta t_1$, $\Delta t_2$ et $\Delta t_3$. La figure 3B correspond à une configuration selon laquelle chaque marqueur fluorescent comporte un terminateur. Durant la période $\Delta t_1$, on mesure, au cours de quatre cycles successifs, le signal $S^*_1$. Durant la période $\Delta t_2$, on mesure, , au cours de quatre cycles successifs, le signal $S^*_2$. Durant la période $\Delta t_3$, on mesure, , au cours de quatre cycles successifs, le signal $S^*_3$.

Le recours à la séquence de calibration permet, une détermination expérimentale des niveaux $S^*_1$, $S^*_2$ et $S^*_3$, et cela sur chaque site de capture, sachant que le nombre de séquences identiques peut varier d'un site à un autre. Les niveaux ainsi établis sont utilisés pour interpréter les signaux de détection détectés lors des cycles pratiqués sur chaque séquence à caractériser Selon une variante, illustrée sur la figure 2D, le signal de détection est caractérisé durant plusieurs périodes de détection successives $\Delta t_{d-1}$, $\Delta t_{d-2}$ et $\Delta t_{d-3}$. Dans un tel mode de réalisation, le recours à une séquence de calibration est également préférable.

Au cours de chaque cycle, l'unité de traitement 41 établit un signal de caractérisation S*, qui caractérise le signal de détection S mesuré aux bornes du circuit de détection 40, le signal de détection résultant d'un courant électrique formé au niveau d'au moins un nanofil. Le signal de caractérisation S* peut comporter une intégration ou une dérivée de l'intensité mesurée par chaque nanofil pris en compte. L'unité de traitement compare la caractérisation à des valeurs mémorisées ou aux valeurs établies durant la période de calibration, chaque valeur étant assignée à un marqueur fluorescent.

Le signal de caractérisation permet d'identifier un marqueur fluorescent parmi plusieurs marqueurs fluorescents dits candidats (dans cet exemple A*, C* et G*), dont les caractéristiques sont connues, en terme d'intensité d'émission ou de décroissance, et cela dans la ou chaque période de détection. Les caractéristiques peuvent être mémorisées dans la mémoire 42. Cela permet d'identifier la base nucléique hybridée au cours du cycle.

Lorsqu'on utilise trois bases marquées, des marqueurs fluorescents convenant à une mise en oeuvre de l'invention sont : DAPI (temps de demi vie 1.88 ns), Hoechst33342 (temps de demi vie 1.05 ns) et Hoeschst 33258 (temps de demi vie 1.22 ns). Les longueurs d'onde conférant une excitation maximale sont respectivement 358 nm, 350 nm et 349 nm.

Le mode de réalisation selon lequel les différents types de bases complémentaires sont mises en oeuvre simultanément est particulièrement adapté à la mise en oeuvre de l'invention. Les marqueurs fluorescents liées à chaque base comportent un terminateur, ce dernier étant éliminé par clivage suite à la formation du signal de caractérisation. Cependant, l'invention peut être mise en oeuvre en exposant les séquences à caractériser à des bases connues en mettant en oeuvre des bains successifs.

On note que les marqueurs fluorescents peuvent faire l'objet d'un clivage au cours de chaque cycle, suite à la formation du signal de caractérisation. Ceci n'est cependant pas impératif.

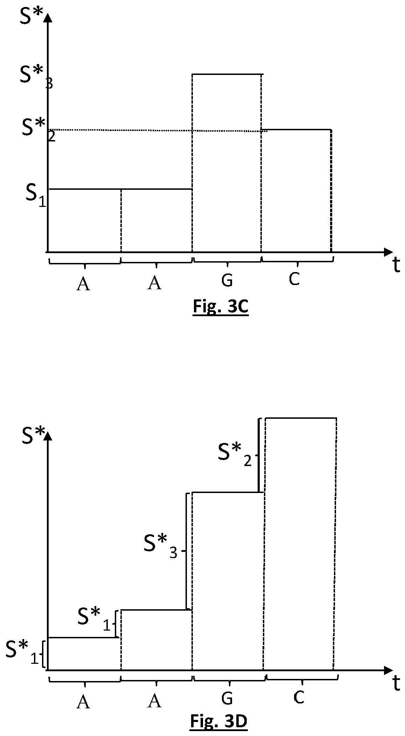

Sur la figure 3C, on a schématisé le signal de caractérisation résultant du séquençage d'une séquence TTCG. Dans cet exemple, le signal de caractérisation correspond à une intégrale du signal de détection durant une période de détection prédéterminée. Chaque base peut être identifiée à partir des signaux $S^*_1$, $S^*_2$ et $S^*_3$ établis durant la calibration. Dans cet exemple, on suppose que suite à chaque détection, le marqueur fluorescent subit un clivage. L'axe des abscisses désigne la base détectée. L'axe des ordonnées correspond au signal de caractérisation.

Sur la figure 3D, on a schématisé le signal de caractérisation résultant de la même séquence. Dans cet exemple, le signal de caractérisation correspond à une intégrale du signal de détection durant une période de détection prédéterminée. Chaque base peut être identifiée à partir des signaux $S^*_1$, $S^*_2$ et $S^*_3$ établis durant la calibration et mémorisés dans la mémoire 42. Dans cet exemple, il n'y a pas de clivage de marqueur fluorescent. L'intensité du signal de fluorescence croît progressivement, sous l'effet de l'accumulation des marqueurs fluorescents liés à des bases hybridées sur la séquence à caractériser.

Le recours à une séquence de calibration peut également être utile lorsque le signal de caractérisation est un signal différentiel, de type dérivée temporelle. Cela peut permettre de calibrer le signal de détection pour les différentes bases. Lorsque le signal de caractérisation est de type dérivée temporelle, il est préférable que les marqueurs fluorescents fassent l'objet d'un clivage suite à la détection de l'hybridation.

D'une façon générale, le recours à la séquence de calibration permet d'établir une réponse du dispositif sur chaque site de capture. Cela permet de prendre en compte les caractéristiques d'émission de fluorescence de chaque marqueur fluorescent, la variabilité du nombre de séquences amplifiées au niveau de chaque site de capture, mais également les éventuelles variabilités de la performance de détection de chaque nanofil.

De préférence, les marqueurs fluorescents sont excités dans une même bande spectrale d'excitation, ce qui permet le recours à une même source de lumière. Selon une alternative, les marqueurs fluorescents sont excités dans des bandes spectrales d'excitation différentes, ce qui peut nécessiter le recours à différentes sources de lumière.

Le recours à des nanofils est particulièrement avantageux. Cela permet d'obtenir un signal exploitable en utilisant un faible nombre de séquences répliquées sur un même site de capture. Cela permet de réduire la taille des sites de capture, relativement à l'art antérieur. On peut ainsi caractériser, sur la même surface de fonctionnalisation, un plus grand nombre de séquences. Les cycles peuvent être mis en oeuvre en parallèle sur chaque site de capture défini sur la surface de fonctionnalisation. La réduction du nombre d'amplifications permet de limiter les inconvénients liés au processus d'amplification des séquences : volume de réactifs nécessaires plus faible, nombre de réplicats plus faible d'où un nombre d'erreurs d'amplification plus faible.

En effet, le circuit de détection permet une mesure de courants très faibles, de l'ordre de quelques dizaines de pA à quelques nA. De plus, les nanofils peuvent être faiblement espacés les uns des autres, typiquement selon un pas spatial inférieur à 500 nm. (contre typiquement 1.1-1.7 $\mu$m pour un détecteur optique CMOS). Cela permet de disposer d'un dispositif présentant un ratio surface utile (i-e la surface de fonctionnalisation) sur volume fluidique élevé.

La bande spectrale d'excitation est de préférence comprise entre 350nm et 550 nm. La bande spectrale de détection de chaque nanofil est de préférence dimensionnée de façon à permettre une détection de la lumière de fluorescence tout en masquant la lumière d'excitation. De façon alternative, les courants de détection, respectivement formés au niveau de chaque nanofil durant l'excitation et l'émission de la lumière de fluorescence, peuvent être temporellement séparés. On tire profit du temps de réponse rapide des nanofils, de l'ordre de la ps, comme décrit dans la littérature Gallo, et al., « Picosecond response times in GaAs/AlGaAs core/shell nanowire-based photodetectors » Appl. Phys. Lett. 2011, 98(24) 241113.

On va à présent décrire différents aspects de conception du dispositif selon l'invention.

Formation des nanofils

Les figures 4A à 4E schématisent des étapes de formation de nanofils à partir d'un substrat 10 de silicium. Les figures 4A à 4E correspondent à une approche dite montante (ou bottom-up). Le substrat 10 est formé de Si, d'orientation (111) et comporte une couche superficielle $10_1$ de $SiO_2$, destinée à former une couche barrière, d'épaisseur 10nm - 15 nm. La couche de $SiO_2$ est recouverte d'une couche $10_2$ de PMMA électrosensible d'épaisseur 45-80 nm. Les couches $10_1$ et $10_2$ font l'objet respectivement d'une photolithographie et d'une gravure, de façon à former des nanopuits isolés les uns des autres, selon un motif prédéterminé. Cf. figure 4A. Les nanopuits débouchent sur le substrat de Si. Une fine couche de métal $10_3$, par exemple de l'or, d'épaisseur 5 à 10 nm est déposée sur la couche de $SiO_2$. Le métal ajouté, en l'occurrence l'or, joue le rôle de catalyseur. Cf. figure 4B. L'excès d'or entre les nanopuits est éliminé par lift of (retrait) de la couche $10_2$ de PMMA. Cf. figure 4C. On obtient ainsi des ilots d'or $10_3$ isolés les uns des autres, aux positions correspondant à la position des nanopuits préalablement formés.

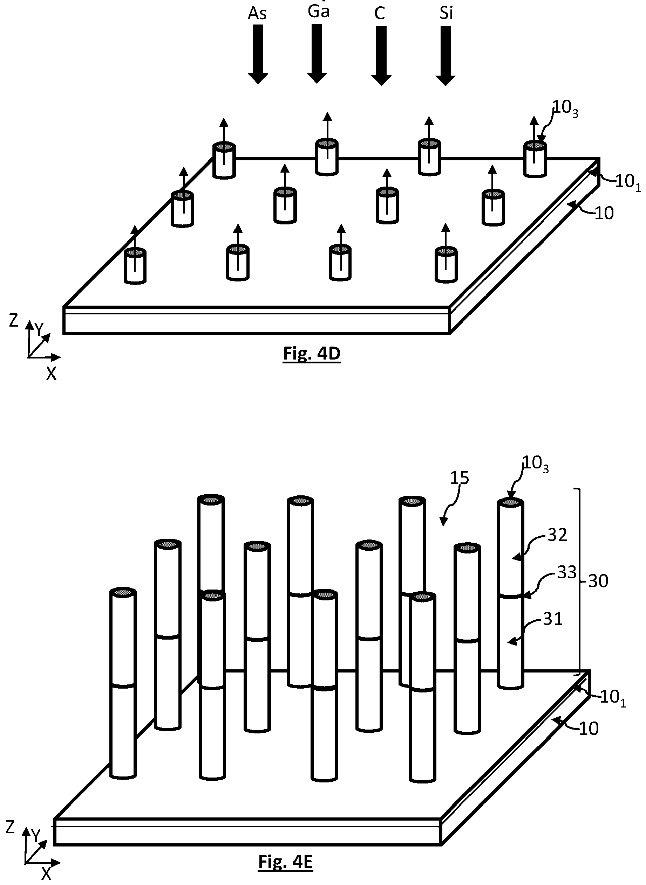

Après chauffage à une température supérieure à 450°C, les ilots forment des gouttes. Les gouttes d'or jouent le rôle de catalyseur. Les nanofils de semi-conducteur sont ensuite formés par épitaxie par jet moléculaire (MBE : molecular beam epitaxy). Cela consiste à envoyer un ou plusieurs jets moléculaires vers le substrat pour réaliser une croissance épitaxiale. Cf. figure 4D. Les jets moléculaires en phase vapeur comportent les espèces chimiques composant le nanofil semi-conducteur ainsi que les espèces dopantes (par exemple Ga et As pour former du GaAs). Les espèces atomiques s'adsorbent et diffusent à la surface du substrat Si sous forme d'adatomes. Les adatomes sont incorporés dans les gouttelettes d'or. Lorsque ces dernières saturent, la nucléation des nanofils se produit tout d'abord à l'interface gouttelette/substrat, puis à l'interface gouttelette/nanofil en formation.

Le procédé n'est pas limité à l'utilisation d'or en tant que catalyseur. D'autres catalyseurs peuvent être utilisés, par exemple Ga ou Sn. Lorsque le catalyseur est Ga et que Ga constitue également un élément constituant le semi-conducteur, on parle de croissance de nanofils autocatalysée.

Ce procédé permet par exemple une croissance de nanofils en utilisant As, Ga ainsi que C et Si respectivement comme dopant p et n, la température de mise en oeuvre étant de 600°C-610°C. Le procédé se poursuit jusqu'à ce que les nanofils atteignent une hauteur prédéterminée. Cf. figure 4E.

Suite à l'étape représentée sur la figure 4E, la couche d'encapsulation 15 est formée entre les nanofils, par exemple par spin-coating. La couche d'encapsulation permet d'isoler électriquement les nanofils les uns des autres, et confère une meilleure tenue mécanique à l'ensemble. Une gravure chimique, plasma et/ou un polissage peut être effectué à l'extrémité des nanofils opposée au substrat, de façon à éliminer les résidus du catalyseur et homogénéiser la hauteur des nanofils et de la couche d'encapsulation 15.

La couche conductrice 22, puis la couche d'interface 23, sont ensuite successivement déposées sur l'ensemble formé par les nanofils et la couche d'encapsulation 15.

Dans le mode de réalisation décrit sur les figures 4A à 4E, la formation des nanopuits sur le substrat 10 permet de maîtriser la position des nanofils. Ainsi, les nanofils peuvent être agencés régulièrement, par exemple selon des motifs de maille respectivement carrée ou hexagonale. Le pas entre deux nanofils adjacents peut être faible, de l'ordre de deux fois le diamètre, ou être plus élevé, par exemple quelques à plusieurs centaines de nm.

Un autre avantage de l'approche montante (ou bottom up), décrite en lien avec les figures 4A à 4E est qu'elle permet un contrôle plus précis de la structure cristalline des nanofils. L'approche montante permet une incorporation maîtrisée de boites quantiques (quantum dots) ou de puits quantiques, en contrôlant leur position et composition, en particulier selon l'axe Z.

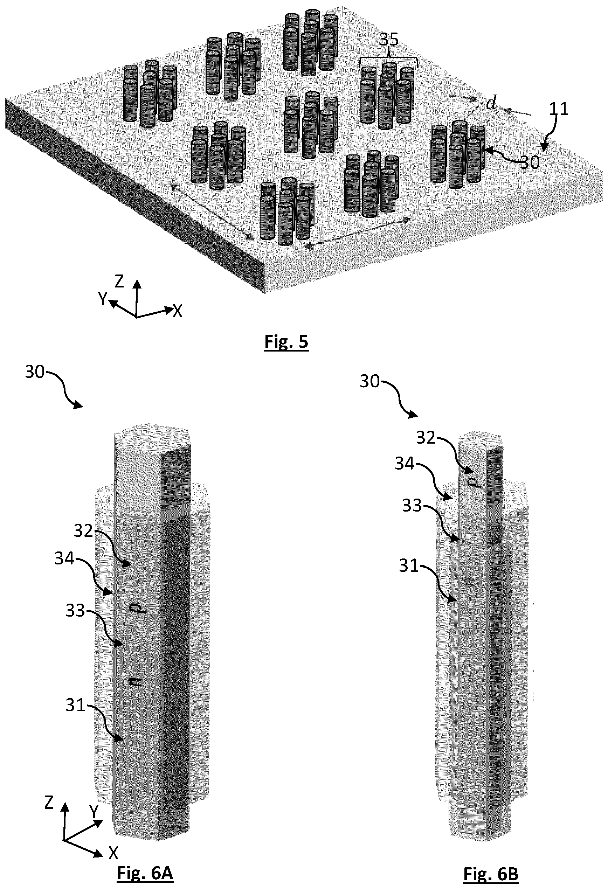

La figure 5 illustre une configuration selon laquelle les nanofils sont agencés en formant des grappes 35, ou clusters. Les nanofils d'un même cluster sont rapprochés les uns des autres, la distance d entre deux nanofils adjacents d'une même grappe étant de préférence supérieure ou égale au diamètre des nanofils. La distance entre deux grappes adjacentes peut être égale ou supérieure à deux fois la distance d.

Lorsque les nanofils sont répartis en clusters, (ou grappes) comme décrit en lien avec la figure 5, les nanofils d'un même cluster sont de préférence raccordés à une même électrode, à la fois sur le substrat 10 et sur la structure multicouche 20. Les nanofils d'un même cluster adressent un même site de capture. Les nanofils d'un même cluster sont ainsi simultanément fonctionnels. Les nanofils non reliés au circuit de détection ne sont pas fonctionnels. La répartition en clusters des nanofils permet de tirer profit de la détection potentielle, par plusieurs nanofils adjacents, de la fluorescence résultant de l'hybridation de bases de même type sur des séquences de même type.

Du fait de la sensibilité élevée de chaque nanofil, le nombre de nanofils composant une même grappe peut être relativement faible. Ainsi, la surface, dans le plan $P_{XY}$, de chaque grappe, est faible, chaque grappe adressant sélectivement une séquence de nucléotides différente d'une autre grappe. Il est ainsi possible de disposer un grand nombre de grappes, adressant respectivement différentes séquences de nucléotides, dans un même dispositif compact.

L'agencement des nanofils en clusters 35 peut être combiné avec une structuration de la surface de fonctionnalisation en nanopuits 27, comme décrit en lien avec la figure 1C. Dans ce cas, chaque nanopuits 27 s'étend face aux nanofils appartenant à un même cluster 35.

Selon une autre possibilité, les nanofils sont obtenus par gravure, selon une approche dite descendante (ou top down). L'approche descendante est décrite dans la demande de brevet FR2114563 déposée le 27/12/2021.

Structure des nanofils

Les figures 6A et 6B représentent d'autres structures de nanofils pouvant être mises en oeuvre dans un dispositif selon l'invention. La figure 6A représente un nanofil similaire aux nanofils précédemment décrits. La jonction 33 est disposée axialement, en s'étendant entre deux parties 31, 32, de dopage différents, espacées l'une de l'autre selon l'axe transversal Z. Dans l'exemple de la figure 6A, une gaine de passivation 34 contourne le nanofil.

Dans l'exemple de la figure 6B, la jonction 33 s'étend radialement entre deux zones de dopage différents. Ainsi, la jonction 33 s'étend autour de l'axe transversal Z, parallèlement à ce dernier. La première partie 31 et la deuxième partie 32 sont séparées radialement, la séparation entre les deux parties correspondant à un rayon de séparation. La première partie s'étend entre l'axe du nanofil et la jonction 33, tandis que la deuxième partie s'étend autour de la jonction 33.

Une structure axiale, est considérée comme avantageuse car elle favorise une incorporation de puits quantiques ou de points quantiques à l'intérieur des nanofils afin d'ajuster le spectre d'absorption.

Une structure radiale permet de disposer d'une jonction 33 s'étendant selon une hauteur importante selon l'axe Z, ce qui permet d'augmenter la sensibilité de détection. De façon optionnelle, la structure radiale représentée sur la figure 6B comporte une gaine annulaire 34 telle que décrite en lien avec la figure 6A.

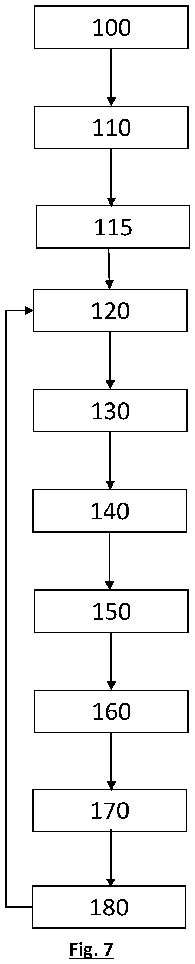

La figure 7 schématise les principales étapes d'un procédé de mise en oeuvre de l'invention.

Etape 100 : disposition d'un échantillon comportant des séquences d'acides nucléiques issus d'une librairie préalablement préparée à partir du brin d'ADN à séquencer, mis au contact de la surface de fonctionnalisation 25.

Etape 110 : capture de séquences d'acides nucléiques par la surface de fonctionnalisation, de préférence au niveau de sites de captures agencés à l'aplomb des nanofils.

Etape 115 : amplification, par exemple par pont, de la séquence d'acides nucléiques capturée dans chaque site. L'étape 115 est optionnelle, mais elle permet d'obtenir, lors de chaque cycle, un signal de détection d'intensité plus élevée, du fait de l'augmentation de la lumière de fluorescence.

Etape 120 : ajout d'un milieu réactionnel, comportant des bases nucléiques marquées par des marqueurs fluorescents et d'une hybridase. De préférence, une seule base nucléique est susceptible d'être hybridée sur chaque séquence. Chaque type de base est marquée par un marqueur fluorescent dit marqueur fluorescent candidat prédéterminé, couplé à un terminateur. Les bases de même type sont marquées par un même marqueur fluorescent candidat.

Etape 130 : rinçage de l'échantillon ;

Etape 140 : illumination de la surface de fonctionnalisation, de façon à entraîner une fluorescence de marqueurs fluorescents hybridés suite à l'étape 120 ;

Etape 150 : détection d'un signal de détection au bornes du circuit de détection.

Etape 160 : caractérisation du signal de détection, de façon à identifier le marqueur fluorescent parmi les marqueurs fluorescents candidats.

Etape 170 : clivage du terminateur, clivage éventuel du marqueur fluorescent, et rinçage de l'échantillon ;

Etape 180 : réitération des étapes 120 à 170, jusqu'au séquençage de chaque séquence de nucléotide capturée au niveau d'un site de capture.

Dans le procédé décrit en lien avec la figure 7, lors de l'étape 120, le milieu réactionnel comporte un mélange de bases. Comme précédemment indiqué, au cours de chaque cycle, on peut prévoir 4 bains successifs, comportant respectivement les 4 types de bases marquées par des marqueurs fluorescents. Ces derniers peuvent alors être identiques ou différents les uns des autres.

Le dispositif tire profit d'un temps de réponse rapide, typiquement de l'ordre de la ns.

On observe que le dispositif ne nécessite pas le recours à des composants optiques volumineux. De plus, la réponse du dispositif est stable, et peu sensibles aux variations environnementales : pH de l'échantillon, température, présence de molécules ou d'ions différents de la biomolécule d'intérêt. Cela est dû au fait que les nanofils ne sont pas au contact de l'échantillon, mais isolés, physiquement et électriquement, de ce dernier par la couche d'interface 23.

Enfin, le dispositif étant basé sur des nanophotodétecteurs, il permet d'obtenir une plateforme d'analyse compacte. Le dispositif peut être obtenu en mettant en oeuvre un procédé de fabrication collective, ce qui permet d'abaisser le coût.

**Revendications**

1. Dispositif (1) d'identification d'un marqueur fluorescent, le marqueur fluorescent étant configuré pour émettre une lumière de fluorescence dans une bande spectrale de fluorescence, le dispositif comportant :

   - un substrat (10), comportant au moins une première électrode ($11_c$) ;
   - une structure multicouches (20), comportant au moins une deuxième électrode ($21_c$) ;
   - des nanofils (30), s'étendant entre la première électrode ($11_c$) et la deuxième électrode ($21_c$), parallèlement à un axe transversal (Z) ;
- une couche d'encapsulation (15) s'étendant autour des nanofils, entre le substrat et la structure multicouches (20), la couche d'encapsulation étant formée d'un matériau isolant ;
- la structure multicouches comportant :

    • une couche conductrice (22), formant chaque deuxième électrode;
    • une couche d'interface (23), électriquement isolante, recouvrant chaque deuxième électrode ($21_c$), chaque deuxième électrode étant interposée entre la couche d'interface et un nanofil, la couche d'interface étant délimitée par une surface de fonctionnalisation (25), la couche d'interface étant configurée pour être disposée entre un échantillon, comportant le marqueur fluorescent, et la deuxième électrode, de telle sorte que la surface de fonctionnalisation forme une interface entre le dispositif et l'échantillon ;
    • la structure multicouche étant telle que la deuxième électrode et la couche d'interface sont transparentes dans une bande spectrale de détection comportant la bande spectrale de fluorescence ;

le dispositif étant tel que :

    - chaque nanofil (30) comporte une jonction (33) de type homojonction, ou une hétérojonction, ou une jonction Schottky entre la première électrode et la deuxième électrode ;
    - la première électrode et la deuxième électrode sont configurées pour être raccordées à un circuit de détection (40);
    - de telle sorte que chaque nanofil forme un nanophotodétecteur de la lumière de fluorescence lorsque le marqueur fluorescent est lié à la surface de fonctionnalisation, la lumière détectée par chaque nanofil induisant un signal électrique de détection dans le circuit de détection ;

le dispositif comportant une unité de traitement, programmée pour :

    • acquérir le signal de détection durant une période temporelle de détection ;
    • déterminer une caractéristique du signal de détection durant la période temporelle de détection ;
    • identifier le marqueur fluorescent en fonction de la caractéristique.

**2.** Dispositif selon la revendication 1, comportant une source de lumière, configurée pour émettre une lumière d'excitation dans la bande spectrale d'excitation du marqueur fluorescent.

**3.** Dispositif selon l'une quelconque des revendications précédentes, dans lequel :

    - la source de lumière est configurée pour émettre la lumière d'excitation durant une période temporelle d'excitation ;
    - la période temporelle de détection est postérieure à la période temporelle d'excitation ;
    - suite à la période temporelle d'excitation, l'intensité de la lumière de fluorescence suit une croissance puis une décroissance ;
    - la caractéristique du signal de détection est représentative de la décroissance de l'intensité de la lumière de fluorescence.

**4.** Dispositif selon l'une quelconque des revendications précédentes, dans lequel la surface de fonctionnalisation est configurée pour capturer un brin formant une chaine d'oligonucléotides.

**5.** Dispositif selon l'une quelconque des revendications précédentes, dans lequel la surface de fonctionnalisation (25) est segmentée en différents sites de capture, chaque site de capture étant configuré pour capturer un brin formant une chaine d'oligonucléotides..

**6.** Dispositif selon la revendication 5 dans lequel :

    - la couche d'interface comporte deux sous-couches ($23_1$, $23_2$), empilées l'une sur l'autre, formant une sous-couche inférieure et une sous-couche supérieure, la sous-couche inférieure étant interposée entre la couche conductrice (22) et la sous-couche supérieure ;
    - la sous-couche supérieure comporte des puits, débouchant dans la sous-couche inférieure, chaque puits étant disposé face à un nanofil, chaque puits formant une partie de la surface de fonctionnalisation ;
    - la surface de fonctionnalisation est segmentée au niveau de chaque puits, de façon que chaque puits forme un site de capture.

**7.** Dispositif selon l'une quelconque des revendications précédentes, comportant plusieurs nanofils, s'étendant entre une même première électrode et une même deuxième électrode, les nanofils formant une grappe de nanofils.

**8.** Dispositif selon la revendication 7, comportant plusieurs grappes de nanofils (35), distantes les unes des autres, de telle sorte qu'un nanofil d'une grappe est plus proche d'un autre nanofil de ladite grappe que d'un autre nanofil d'une autre grappe, les nanofils d'une même grappe s'étendant entre une même première électrode et une même deuxième électrode.

9. Dispositif selon l'une quelconque des revendications précédentes, comportant plusieurs nanofils, le dispositif étant tel que :

- plusieurs premières électrodes sont formées sur le substrat, et plusieurs deuxièmes électrodes sont formées sur la structure multicouche, chaque nanofil s'étendant entre une première électrode et une deuxième électrode ;
- chaque première électrode est reliée à une première unité d'adressage ($40_X$), configurée pour sélectionner au moins une première électrode ;
- chaque deuxième électrode est reliée à une deuxième unité d'adressage ($40_Y$), configurée pour sélectionner au moins une deuxième électrode ;
- de telle sorte que le circuit de détection (40) détecte un courant de détection induit par chaque nanofil s'étendant entre la première électrode et la deuxième électrode sélectionnées.

10. Procédé d'identification d'un marqueur fluorescent à l'aide d'un dispositif selon l'une quelconque des revendications précédentes, le marqueur fluorescent étant susceptible d'émettre une lumière de fluorescence, dans la bande spectrale de détection, lorsqu'il est illuminé par la lumière d'excitation, la surface de fonctionnalisation étant configurée pour capturer un brin d'acide nucléique, le procédé comportant :

a) disposition d'un échantillon, comportant des acides nucléiques, au contact de la surface de fonctionnalisation ;
b) capture d'au moins un brin d'acide nucléique sur la surface de fonctionnalisation ;
c) ajout de bases nucléiques dans l'échantillon, au moins deux bases nucléiques différentes étant marquées par deux marqueurs fluorescents différents, l'échantillon comportant des principes actifs configurés pour permettre une hybridation d'une base nucléique sur le brin d'acide nucléique capturé sur la surface de fonctionnalisation ;
d) exposition de la surface de fonctionnalisation à une lumière d'excitation, dans une bande spectrale d'excitation d'au moins un marqueur fluorescent, ;
e) suite à l'étape d), détection d'un signal de détection aux bornes du circuit de détection, durant une période temporelle de détection ;
f) en fonction du signal de détection détecté lors de l'étape e), identification du marqueur fluorescent ;
g) réitération des étapes c) à f) de façon à hybrider progressivement des bases nucléiques le long du brin d'acide nucléique.

11. Procédé selon la revendication 10, dans lequel l'étape b) comporte une amplification de chaque brin d'acide nucléique capturé.

12. Procédé selon l'une quelconque des revendications 10 ou 11, dans lequel les étapes b) à g) sont mises en oeuvre sur différents sites de capture répartis sur la surface de fonctionnalisation.

13. Procédé selon la revendication 12, dans lequel l'étape f) comporte :

- détermination d'une caractéristique du signal de détection lors de la période temporelle de détection ;
- identification du marqueur fluorescent en fonction de la caractéristique.

14. Procédé selon l'une quelconque des revendications 10 à 13, dans lequel :

- préalablement à l'étape a), chaque séquence de nucléotides est reliée à une séquence de calibration connue ;
- les étapes c) à f) sont mises en oeuvre de façon à hybrider les bases de la séquence de calibration, les étapes c) à f) formant une phase de calibration ;
- le signal de détection obtenu lors de chaque étape e) de la phase de calibration est utilisé pour calibrer une réponse du dispositif aux bases de la séquence de calibration.

15. Procédé selon l'une quelconque des revendications 10 à 14, comportant, suite à l'étape g), une étape h) d'identification de la base d'acide nucléique hybridée.

16. Procédé selon la revendication 15, dans lequel

- suite à l'étape h), le marqueur fluorescent est clivé et l'échantillon est rincé ;
- suite au rinçage, les étapes c) et h) sont réitérées, de manière à identifier une séquence de nucléotides formant le brin d'acide nucléique capturé.

**Fig. 1A**

**Fig. 1B**

**Fig. 1C**

**Fig. 1D**

**Fig. 1E**

**Fig. 2A**

**Fig. 2B**

$\Delta t_d$

S*

G*

C*

A*

t

**Fig. 2C**

$i$

$\Delta t_{d-1}$

$\Delta t_{d-2}$

$\Delta t_{d-3}$

G*

$i_3$

C*

$i_2$

$I_{max}$

A*

$i_1$

ex

$\Delta t_{ex}$

t

**Fig. 2D**

**Fig. 3A**

**Fig. 3B**

**Fig. 3C**

**Fig. 3D**

**Fig. 4A**

**Fig. 4B**

**Fig. 4C**

As    Ga    C    Si

$10_3$

$10_1$

10

Z Y X

**Fig. 4D**

$10_3$

15

32

33    30

31

$10_1$

10

Z Y X

**Fig. 4E**

**Fig. 5**

**Fig. 6A**

**Fig. 6B**

**Fig. 7**

**EP 4 484 932 A1**

Europäisches
Patentamt

European
Patent Office

Office européen
des brevets

# RAPPORT DE RECHERCHE EUROPEENNE

Numéro de la demande

EP 24 18 4503

## DOCUMENTS CONSIDERES COMME PERTINENTS

| Catégorie | Citation du document avec indication, en cas de besoin, des parties pertinentes | Revendication concernée | CLASSEMENT DE LA DEMANDE (IPC) |
|---|---|---|---|
| Y | MARIA SPIES ET AL: "Nanowire photodetectors based on wurtzite semiconductor heterostructures", ARXIV.ORG, CORNELL UNIVERSITY LIBRARY, 201 OLIN LIBRARY CORNELL UNIVERSITY ITHACA, NY 14853, 27 avril 2019 (2019-04-27), XP081268008, DOI: 10.1088/1361-6641/AB0CB8 | 1-7,9-16 | INV. G01N21/64 C12Q1/6869 |
| A | * abrégé * <br> * titre * <br> * alinéas [0003], [0006] * <br> * figure 8 * <br> ----- | 8 | |
| Y | US 2011/253982 A1 (WANG DELI [US] ET AL) 20 octobre 2011 (2011-10-20) * alinéas [0009] - [0011], [0020] - [0043] * * figures 4, 6 * <br> ----- | 1-7,9-16 | |
| Y | LI ZIYUAN ET AL: "Review on III-V Semiconductor Nanowire Array Infrared Photodetectors", ADVANCED MATERIALS TECHNOLOGIES , vol. 8, no. 13 4 avril 2023 (2023-04-04), XP093124564, DE ISSN: 2365-709X, DOI: 10.1002/admt.202202126 Extrait de l'Internet: URL:https://onlinelibrary.wiley.com/doi/pdf/10.1002/admt.202202126 * abrégé * * titre * * figures 2, 5, 8 * <br> ----- <br> -/-- | 1-7,9-16 | DOMAINES TECHNIQUES RECHERCHES (IPC) G01N |

Le présent rapport a été établi pour toutes les revendications

| Lieu de la recherche | Date d'achèvement de la recherche | Examinateur |
|---|---|---|
| Munich | 4 novembre 2024 | Brauer, Jan |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

EPO FORM 1503 03.82 (P04C02)

page 1 de 2

Europäisches
Patentamt

European
Patent Office

Office européen
des brevets

# RAPPORT DE RECHERCHE EUROPEENNE

Numéro de la demande

EP 24 18 4503

## DOCUMENTS CONSIDERES COMME PERTINENTS

| Catégorie | Citation du document avec indication, en cas de besoin, des parties pertinentes | Revendication concernée | CLASSEMENT DE LA DEMANDE (IPC) |
|---|---|---|---|
| Y | US 2022/099575 A1 (ROTHBERG JONATHAN M [US] ET AL) 31 mars 2022 (2022-03-31) | 1-7,9-16 | |
| A | * alinéas [0013], [0207] - [0218], [0237], [0238], [0346], [0427], [0435], [0491] - [0566] * <br> * figures 3,5,10 * <br> - - - - - | 8 | |

**DOMAINES TECHNIQUES RECHERCHES (IPC)**

Le présent rapport a été établi pour toutes les revendications

| Lieu de la recherche | Date d'achèvement de la recherche | Examinateur |
|---|---|---|
| Munich | 4 novembre 2024 | Brauer, Jan |

CATEGORIE DES DOCUMENTS CITES

X : particulièrement pertinent à lui seul
Y : particulièrement pertinent en combinaison avec un
    autre document de la même catégorie
A : arrière-plan technologique
O : divulgation non-écrite
P : document intercalaire

T : théorie ou principe à la base de l'invention
E : document de brevet antérieur, mais publié à la
    date de dépôt ou après cette date
D : cité dans la demande
L : cité pour d'autres raisons

& : membre de la même famille, document correspondant

EPO FORM 1503 03.82 (P04C02)

page 2 de 2

## ANNEXE AU RAPPORT DE RECHERCHE EUROPEENNE
## RELATIF A LA DEMANDE DE BREVET EUROPEEN NO.

EP 24 18 4503

La présente annexe indique les membres de la famille de brevets relatifs aux documents brevets cités dans le rapport de recherche européenne visé ci-dessus.
Lesdits members sont contenus au fichier informatique de l'Office européen des brevets à la date du
Les renseignements fournis sont donnés à titre indicatif et n'engagent pas la responsabilité de l'Office européen des brevets.

04-11-2024

| Document brevet cité au rapport de recherche | Date de publication | Membre(s) de la famille de brevet(s) | Date de publication |
|---|---|---|---|
| US 2011253982 A1 | 20-10-2011 | US 2011253982 A1 | 20-10-2011 |
| | | WO 2010062644 A2 | 03-06-2010 |
| US 2022099575 A1 | 31-03-2022 | AU 2015300779 A1 | 16-03-2017 |
| | | AU 2021203568 A1 | 01-07-2021 |
| | | AU 2023210629 A1 | 24-08-2023 |
| | | BR 112017002501 A2 | 05-12-2017 |
| | | CA 2957546 A1 | 11-02-2016 |
| | | CN 106796176 A | 31-05-2017 |
| | | CN 112903638 A | 04-06-2021 |
| | | CN 112903639 A | 04-06-2021 |
| | | EP 3194934 A1 | 26-07-2017 |
| | | EP 4421188 A2 | 28-08-2024 |
| | | JP 6930911 B2 | 01-09-2021 |
| | | JP 2017525958 A | 07-09-2017 |
| | | JP 2021193375 A | 23-12-2021 |
| | | KR 20170041850 A | 17-04-2017 |
| | | KR 20220165286 A | 14-12-2022 |
| | | US 2016084761 A1 | 24-03-2016 |
| | | US 2016370291 A1 | 22-12-2016 |
| | | US 2016370292 A1 | 22-12-2016 |
| | | US 2017299518 A1 | 19-10-2017 |
| | | US 2018120229 A1 | 03-05-2018 |
| | | US 2019212265 A1 | 11-07-2019 |
| | | US 2020088639 A1 | 19-03-2020 |
| | | US 2022099575 A1 | 31-03-2022 |
| | | WO 2016023011 A1 | 11-02-2016 |

EPO FORM P0460

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- US 2011253982 A
- US 2022099575 A

- FR 2114563

**Littérature non-brevet citée dans la description**

- **SING et al.** Silicon nanowire optical rectangular waveguide biosensor for DNA Hybridization. *IEEE Photonics Technology Letters,* 2018, vol. 30 (12), 1123-1126
- **IRRERA et al.** New génération of ultrasensitive label-free optical Si nanowire-based biosensors. *ACS Photonics,* 2018, vol. 5 (2), 471-479
- **ZIYUAN LI et al.** *Review on III V Semiconductor nanowire array infrared photodetectors*

- **VALENTE et al.** Light-Emitting GaAs Nanowires on a Flexible Substrate. *Nano Lett,* 2018, vol. 18 (7), 4206-4213
- **MOKKAPATI et al.** Optical design of nanowire absorbers for wavelength selective photodetectors. *Sci. Rep.,* 2015, vol. 5, 15339
- **GALLO et al.** Picosecond response times in GaAs/AlGaAs core/shell nanowire-based photodetectors. *Appl. Phys. Lett.,* 2011, vol. 98 (24), 241113